# EUROPEAN PATENT APPLICATION

(11) **EP 2 292 270 A1**
(43) Date of publication of application: **09.03.2011**
(21) Application number: 08791872.8
(22) Date of filing: 30.07.2008
(51) Int. Cl.: A61K 47/34, A61K 31/7088, A61K 47/24

(54) **DRUG CARRIER**

(30) Priority: 19.06.2008 JP 2008160231
(71) Applicant: Nippon Shinyaku Co., Ltd., Kyoto-shi Kyoto 601-8550 (JP)
(72) Inventor: SONOKE, Satoru, Tsukuba-shi Ibaraki 305-0061 (JP); UEDA, Toshihiro, Tsukuba-shi Ibaraki 305-0003 (JP)
(74) Representative: Vossius & Partner
(86) International application number: PCT/JP2008/063641
(87) International publication number: WO 2009/153888

(57) **Abstract**

A drug carrier **characterized by** mainly containing a polyethylene-glycol-modified phospholipid and a cationic lipid and containing the polyethylene-glycol-modified phospholipid in a concentration within a specific range. The drug carrier, which is of the in-blood residence type, is **characterized by** comprising a polyethylene-glycol-modified phospholipid represented by the following general formula (I) or a pharmaceutically acceptable salt thereof: 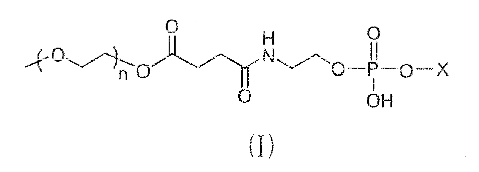 [wherein X represents the following (II) or (III) and n is an integer of 30-150] 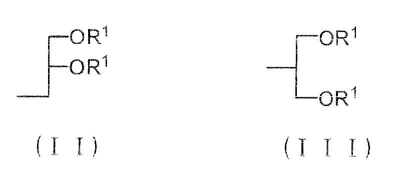 [wherein R¹ represents a saturated linear C₁₇₋₂₂ fatty acid residue] and 2-O-(2-diethylaminoethyl)carbamoyl-1,3-0-dioleoylglylcerol. It is further **characterized in that** the polyethylene-glycol-modified phospholipid represented by the general formula (I) is contained in an amount of 30-50 wt.% based on the total amount of the lipids in the drug carrier.

## Description

### Technical Field

The present invention relates to a novel long-circulating drug carrier.

### Background Art

Attention has been focused recently on nucleic acid medicines such as a synthetic double-stranded RNA (such as poly(I)-poly(C)), a short interfering RNA (siRNA) utilizing RNA interference (RNAi), a microRNA (miRNA), a short hairpin RNA (shRNA), an antisense DNA, and an antisense RNA, which have been actively studied. Such a nucleic acid medicine is hardly delivered to a tissue with a lesion even when the medicine is systemically administered independently in the body through, for example, a vein. Therefore, the nucleic acid medicine needs, for example, administering after it is incorporated in an appropriate carrier or topically administering to a tissue with a lesion.

Examples of a drug carrier for delivering the nucleic acid medicine to a tissue with a lesion include cationic liposomes such as LIPOFECTIN (registered trademark), LIPOFECTAMINE 2000 (registered trademark), and OLIGOFECTAMINE (registered trademark), and a cationic liposome (hereinafter referred to as "Compound A liposome") containing 2-O-(2-diethylaminoethyl)carbamoyl-1,3-O-dioleoylglycerol (hereinafter referred to as "Compound A") and a phospholipid as essential components (see, for example, Patent document 1). Since such cationic liposomes tend to accumulate easily in the liver and spleen when administered systemically through, for example, a vein, it is expected to apply the cationic liposomes as a therapeutic agent for liver cancer or hepatitis by incorporating a nucleic acid medicine in the cationic liposomes. It is actually reported that a complex of Compound A liposome with a synthetic double-stranded RNA such as poly (I)-poly (C) is effective in the treatment of liver cancer or hepatitis (see, for example, Patent document 2, Patent document 3, Non-paten document 1, and Non-patent document 2).
The cationic liposomes are useful as a carrier for accumulating a nucleic acid medicine in the liver or the like. However, it is not sufficient as a carrier for use in delivering a nucleic acid medicine to a tissue (such as lung, kidney, pancreas, or heart) other than the liver or spleen as well as achieving prolonged circulation in blood.

It is reported that by modifying a lipid constituting a liposome modified with polyethylene glycol, the uptake in the reticuloendothelial system is suppressed, and therefore, a circulating property in blood is improved (see, for example, Non-patent document 3).
However, the lipid modified with polyethylene glycol may decrease an efficacy of a medicine incorporated in the liposome with an increase in the content of the lipid. Thus, it is important to add the lipid in a minimum amount capable of having the long-circulating property (see, for example, Patent document 4).

It is reported that distearoyl phosphatidyl ethanolamine modified with polyethylene glycol as a component of a liposome makes a circulation time most prolonged in an amount of about 4 mol% in the total lipids constituting the liposome (see, for example, Non-patent document 3).
Meanwhile, it is also reported that a lipid modified with polyethylene glycol blended in an amount of about 10 or 15 mol% makes a circulation time prolonged (see, for example, Patent documents 5 to 9). According to the Patent documents 5 to 9, depending on the difference in the structure of a lipid modified with polyethylene glycol or a cationic lipid to be used as a component of a liposome, the obtained prolongation effect on the circulation time or the degree of expression of drug efficacy varies.

Patent document 1: WO 94/19314 A1
Patent document 2: WO 99/20283 A1
Patent document 3: WO 99/48531 A1
Patent document 4: WO 2005/051351 A2
Patent document 5: WO 2006/074546 A1
Patent document 6: WO 2006/007712 A1
Patent document 7: WO 2005/120152 A2
Patent document 8: CA 2271582 A1
Patent document 9: US 2004/0166150 A1
Non-patent document 1: Kazuko Hirabayashi, et al., Cancer Research, 1999, Vol. 59, pp. 4325-4333
Non-patent document 2: Kazuko Hirabayashi, et al., Oncology Research, 1999, Vol. 11, pp. 497-504
Non-patent document 3: Tatsuhiro Ishida, et al., Riposomu Oyo no Shintenkai, 2005, June, pp. 526-538

### Disclosure of the Invention

### Problems that the Invention is to Solve

A chief object of the present invention is to provide a drug carriers which is a long-circulating drug carrier mainly containing a polyethylene glycol-modified phospholipid and Compound A, wherein the polyethylene glycol-modified phospholipid is contained at a concentration within a specific range, and a pharmaceutical composition containing the drug carrier incorporating a medicine.

### Means for Solving the Problems

After the present inventors made intensive studies, they found that in a drug carrier containing a polyethylene glycol-modified phospholipid having a specific structure and Compound A as essential components, the polyethylene glycol-modified phospholipid gives the drug carrier a long-circulating property, at a high concentration of from 30 to 50 wt% in the total weight of the lipids in the drug carrier, and also allows a medicine contained thy drug carrier to exhibit am efficacy in vivo. As a result, the present invention has been completed.

The present invention includes, for example, the following inventions described in 1 and 2.
1. A long-circulating drug carrier, comprising a polyethylene glycol-modified phospholipid represented by the following general formula (I) (hereinafter simply referred to as "PEG-modified phospholipid") or a pharmaceutically acceptable salt thereof: 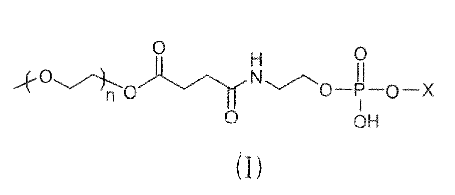 [wherein X represents the following (II) or (III); and n represents an integer of 30 to 150], 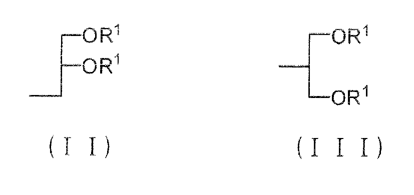 [wherein R¹ represents a saturated linear fatty acid residue having 17 to 22 carbon atoms] and Compound A, wherein the PEG-modified phospholipid represented by the above general formula (I) is contained in an amount within a range from 30 wt% to 50 wt% in the total weight of the lipids in the drug carrier (hereinafter referred to as "the carrier of the present invention")_{.}
2. A pharmaceutical composition, comprising the carrier of the present invention which incorporates a medicine (hereinafter referred to as "the composition of the present invention").

Examples of the saturated linear fatty acid residue having 17 to 22 carbon atoms represented by R¹ include stearoyl, arachidoyl, and behenoyl. Among these, a saturated linear fatty acid residue having 17 to 20 carbon atoms is preferable and stearoyl is more preferable.

n is an integer within a range from 30 to 150, preferably an integer within a range from 30 to 100, more preferably an integer within a range from 30 to 65.

The PEG-modified phospholipid can be used as a free acid as such, however, it can be formed into the form of a pharmaceutically acceptable salt buy a conventional method and used.
The pharmaceutically acceptable salt is not particularly limited, but, examples thereof include sodium salt and potassium salt. Among these, sodium salt is particularly preferable.

Preferable examples of the PEG-modified phospholipid include 1,3-distearoylglycero-2-phosphatidyl-N- (methoxy-polyethylene-glycol-succinyl)ethanolamine and N-(methoxy-polyethylene-glycol-succinyl)distearoylphosphatidylethanol amine.

### Brief Description of the Drawings

[Fig. 1] Fig. 1 shows the mass spectrum of the PEG-modified phospholipid synthesized in Production Example 3.

[Fig. 2] Fig. 2 shows the mass spectrum of the PEG-modified phospholipid used in Production Examples 12 to 15.

[Fig. 3] Fig. 3 shows the time-dependent change in the circulating property in plasma. The vertical axis represents the distribution ratio (% of dose), and the horizontal axis represents the time period (hour) after administration.

[Fig. 4] Fig. 4 shows the circulating property in plasma at 24 hours after administration. The vertical axis represents the distribution ratio (% of dose), and the horizontal axis represents the content (wt%) of the PEG-modified phospholipid used.

[Fig. 5] Fig. 5 shows the circulating property in plasma at 24 hours after administration. The vertical axis represents the distribution ratio (% of dose), and the horizontal axis represents the content (wt%) of the PEG-modified phospholipid used.

[Fig. 6] Fig. 6 shows the circulating property in plasma. The vertical axis represents the distribution ratio (% of dose), and the horizontal axis represents the time period (hour) after administration of the pharmaceutical composition.

[Fig. 7] Fig. 7 shows the circulating property in plasma. The vertical axis represents the distribution ratio (% of dose), and the horizontal axis represents the time period (hour) after administration of the pharmaceutical composition.

[Fig. 8] Fig. 8 shows the delivering property to the liver. The vertical axis represents the distribution ratio (% of dose), and the horizontal axis represents the time period (hour) after administration of the pharmaceutical composition.

[Fig. 9] Fig. 9 shows the delivering property to the spleen. The vertical axis represents the distribution ratio (% of dose), and the horizontal axis represents the time period (hour) after administration, of the pharmaceutical composition.

[Fig. 10] Fig. 10 shows the delivering property to the lung. The vertical axis represents the distribution ratio (% of dose), and the horizontal axis represents the time period (hour) after administration of the pharmaceutical composition.

[Fig. 11] Fig. 11 shows the delivering property to the kidney. The vertical axis represents the distribution ratio (% of dose), and the horizontal axis represents the time period (hour) after administration of the pharmaceutical composition.

[Fig. 12] Fig. 12 shows the delivering property to peripheral tissues of cancer. The vertical axis represents the concentration (µg/g) of the drug carrier, and the horizontal axis represents the time period (hour) after administration of the pharmaceutical composition.

[Fig. 13] Fig. 13 shows the delivering property to cancerous nodes. The vertical axis represents the concentration (µg/g) of the drug carrier, and the horizontal axis represents the time period (hour) after administration of the pharmaceutical composition.

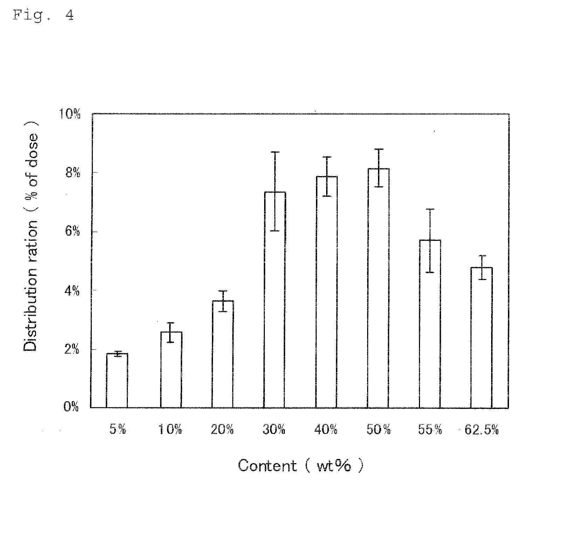

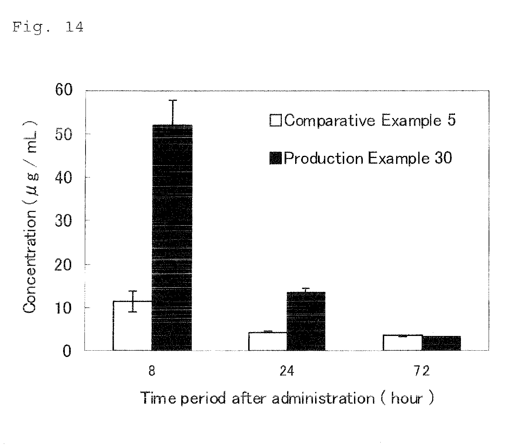

[Fig. 4] Fig. 14 shows the delivering property to plasma. The vertical axis represents the concentration (µg/mL) of the drug carrier, and the horizontal axis represents the time period (hour) after administration of the pharmaceutical composition.

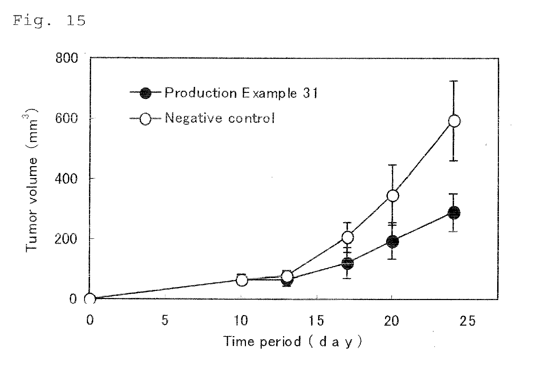

[Fig. 15] Fig. 15 shows the antitumor effect. The vertical axis represents the tumor volume (mm³), and the horizontal axis represents the time period (day) after implantation of cancer cells.

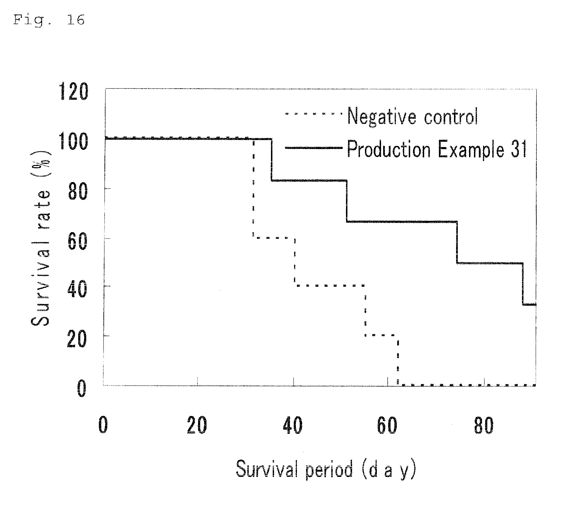

[Fig. 16] Fig. 16 shows the antitumor effect in the case of continuous administration of the composition of the present invention. The vertical axis represents the survival rate (%), and the horizontal axis represents the survival period (day) after implantation of cancer cells.

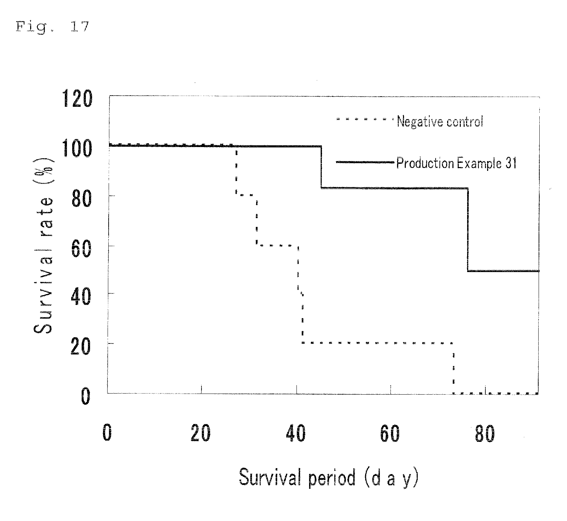

[Fig. 17] Fig. 17 shows the antitumor effect in the case of intermittent administration of the composition of the present invention. The vertical axis represents the survival rate (%), and the horizontal axis represents the survival period (day) after implantation of cancer cells.

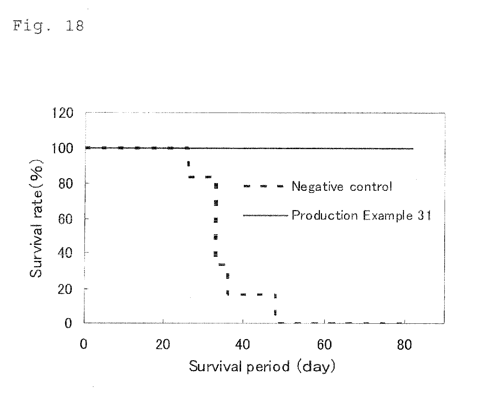

[Fig. 18] Fig. 18 shows the antitumor effect. The vertical axis represents the survival rate (%), and the horizontal axis represents the survival period (day) after implantation of cancer cells.

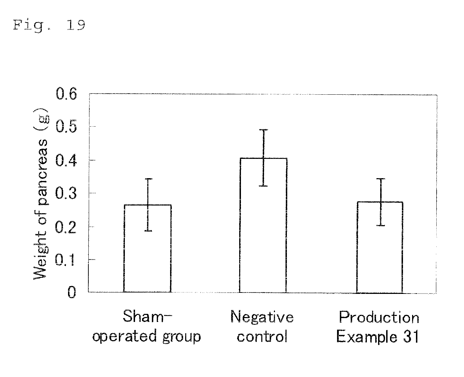

[Fig. 19] Fig. 19 shows the antitumor effect. The vertical axis represents the weight of the pancreas (g).

### Best Mode for Currying Out the Invention

I. Method for producing PEG-modified phospholipid

A PEG-modified phospholipid, (Ia) in which X is the above formula (II) can be produced by reacting an amine derivative represented by the following general formula (1a) with a PEG derivative represented by the following general formula (2) in the presence of an appropriate base.
A solvent to be used in the reaction with the PEG derivative represented by the following general formula (2) is not particularly limited unless it is involved in the reaction, and, examples thereof include dichloromethane, dimethoxyethane, and a mixed liquid thereof. Examples of the base include triethylamine, pyridine, and an aqueous solution of sodium hydrogen carbonate. The reaction temperature is appropriately within a range from 0°C to 50°C. The reaction time varies depending on the type of raw material to be used and the reaction temperature. In general, the reaction time is appropriately within a range from 1 hour to 30 hours.

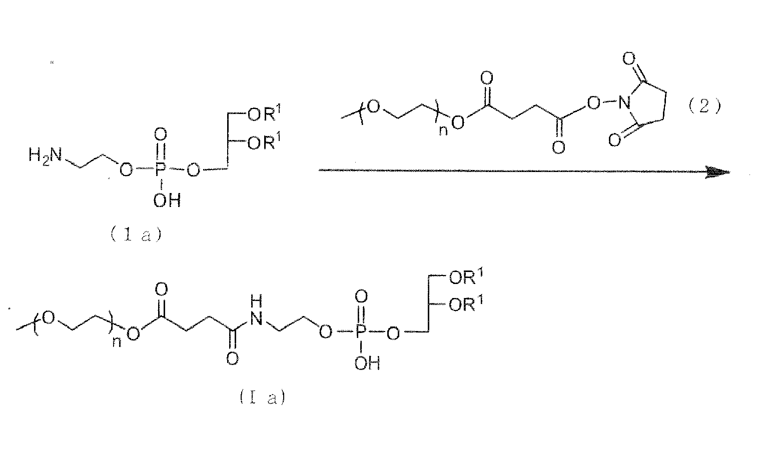

(In the formula, n and R¹ are the same as defined above.)

A PEG-modified phospholipid (Ib) in which X is the above formula (III) can be produced by deprotecting a protecting group (R²) for an amino group and a protecting group (R³) for phosphoric acid of an amine derivative represented by the following general formula (1b) by a conventional method, and then reacting the amine derivative with a PEG derivative represented by the above general formula (2) in the presence of an appropriate base.
Deprotection of R² and R³ can be performed similtaneously or stepwise. Examples of a reagent for deprotecting R² include acids such as trifluoroacetic acid, acetic acid, and hydrochloric acid Examples of a reagent for deprotecting R³ include a mixed liquid of pyridine, triethylamine, and water (3:1:1); an acetonitrile solution of triethylamine; a 50% aqueous dioxane solution of pyridine-2-carboxaldoxime and N¹,N¹,N³,N³-tetramethylguanidine; and acids such as trifluoroacetic acid, acetic acid, and hydrochloric acid.
A solvent to be used in the reaction with the PEG derivative represented by the above general formula (2) is not particularly limited unless it is involved in the reaction, and, examples thereof include dichloromethane, dimethoxyethane, and a mixed liquid thereof. Examples of the base include triethylamine, pyridine, and an aqueous solution of sodium hydrogen carbonate. The reaction temperature is appropriately within a range from 0°C to 50°C. The reaction time varies depending on the type of raw material to be used and the reaction temperature. In general, the reaction time is appropriately within a range from 1 hour to 30 hours.

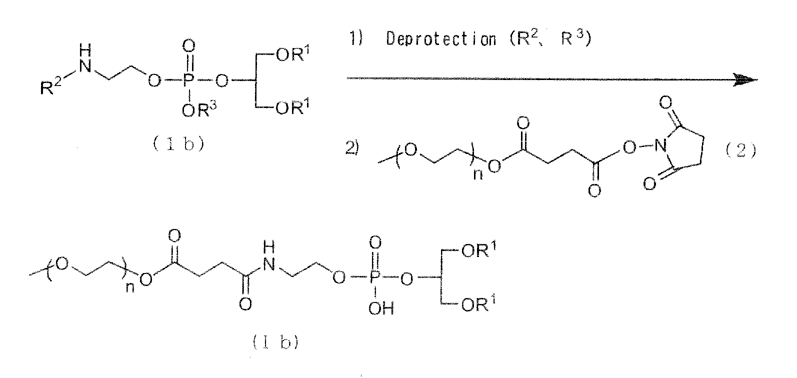

(In the formula, n and R¹ are the same as defined above. R² represents a protecting group for an amino group. The protecting group is not particularly limited, and, examples thereof include tert-butyloxycarbonyl and benzyloxycarbonyl. R³ represents a protecting group for phosphoric acid. The protecting group is not particularly limited, and, examples thereof include methyl, cyanoethyl, and tert-butyl.

The amine derivative represented by the above general formula (1a) can be produced according to the method described in the document (J.Am. Chem. Soc., 1993, 115, pp. 10487-10491) using phosphatidylcholine represented by the following general formula (3), aminoethanol represented by the following general formula (4), and phospholipase D.

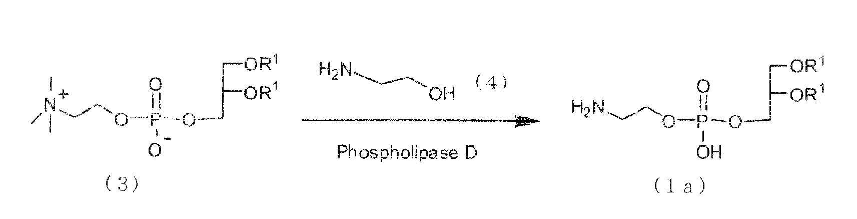

(In the formula, R¹ is the same as defined above.)

The amine derivative represented by the above general formula (1b) can be produced by reacting an amidite compound represented by the following general formula (5) with aminoethanol represented by the following general formula (6) in the presence of an appropriate activating agent, and then oxidizing the resulting product with an appropriate oxidizing agent.
Examples of the activating agent include tetrazole and 5-phenyl-1H-tetrazole. Examples of the oxidizing agent include an iodine solution (0.1 M iodine/tetrahydrofuran : pyridine: water = 7:1:2) and a tert-butyl hydroperoxide solution. The reaction temperature is appropriately within a range from 0°C to 50°C. A solvent to be used is not particularly limited unless it is involved in the reaction, and, examples thereof include acetonitrile and dichloromethane. The reaction time varies depending on the type of raw material to be used and the reaction temperature. In general, the reaction time is appropriately within a range from 1 hour to 30 hours.

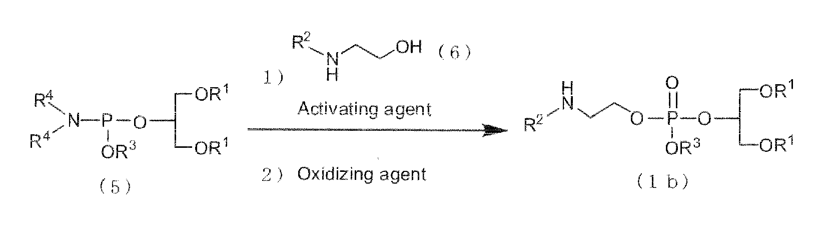

(In the formula, R¹, R², and R³ are the same as defined above. R⁴ represents alkyl. The alkyl is not particularly limited, and, examples thereof include methyl, ethyl, n-propyl, and isopropyl.)

The amidite represented by the above general formula (5) can be produced by converting an alcohol represented by the following general formula (7) to an amidite in the presence of an appropriate activating agent.
Examples of the activating agent include diisopropylammonium tetrazolide, tetrazole, 5-phenyl-1H-tetrazole, and diisopropylethylamine. Examples of a reagent to be used in the conversion to an amidite include bis(N,N-diisopropylamino)cyanoethylphosphite, 2-cyanoethyl N,N-diisopropylchlorophosphoroamidite, and tert-butyl tetraisopropylphosphoroamidite. A solvent to be used is not particularly limited unless it is involved in the reaction, and, examples thereof include acetonitrile and dichloromethane. The reaction temperature is appropriately within a range from 0°C to 50°C. The reaction time varies depending on the type of raw material to be used and the reaction temperature. In general, the reaction time is appropriately within a range from 1 hour to 30 hours.

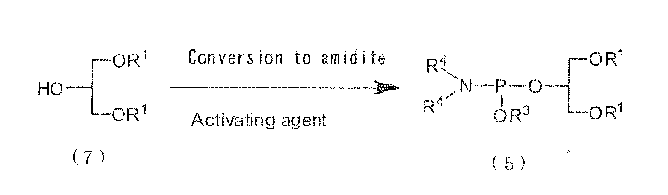

(In the formula, R¹, R³, and R⁴ are the same as defined above.)

The alcohol represented by the above general formula (7) can be produced according to a method described in a document (for example, The Journal of Organic Chemistry, 1970, vol. 35, pp. 2082-2083) using dihydroxyacetone dimer (8). Examples of a condensing agent include N,N'-dicyclohexylcarbodiimide, 1-ethyl-3-(3-dimethylaminopropyl)carbodiimide, and 1-hydroxybenzotriazole. Examples of a reducing agent include sodium borohydride.

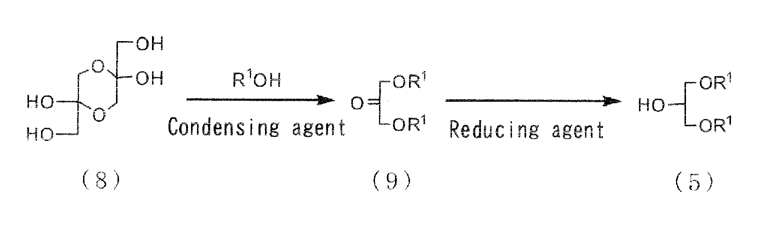

(In the formula, R¹ is the same as defined above.)

II. The carrier of the present invention

The carrier of the present invention contains a PEG-modified phospholipid and Compound A as essential components. Specifically, the carrier of the present invention can take the form of a liposome, a fat emulsion, or the like. Examples of the form of a liposome include a multilamellar vesicle and a unilamellar vesicle.

Compound A can be synthesized by the method described in WO 94/19314.

The blending amount of the PEG-modified phospholipid in the carrier of the present invention is appropriately within a range from 30 wt% to 50 wt%, preferably within a range from 40 wt% to 50 wt% in the total weight of the lipids in the carrier of the present invention.

As for the blending ratio between the PEG-modified phospholipid and Compound A in the carrier of the present invention, the ratio of Compound A is appropriately within a range from 0.2 to 20 parts by weight per 1 part by weight of the PEG-modified phospholipid, preferably within a range from 0.5 to 10 parts by weight, more preferably within a range from 0.7 to 1.3 parts by weight.

To the carrier of the present invention, a phospholipid can additionally be added other than the PEG-modified phospholipid and Compound A which are the essential components. The phospholipid is not particularly limited insofar as it is a pharmaceutically acceptable phospholipid, examples thereof include phosphatidylcholine, phosphatidylethanolamine, phosphatidylinositol, phosphatidylserine, sphingomyelin, lecithin, dipalmitoylphosphatidylcholine, distearoylphosphatidylcholine, and dipalmitoylphosphatidylglycerol. These can be used singly or in combination of two or more thereof. Among these, 1-palmitoyl-2-oleoyl-sn-glycero-3-phosphocholine, phosphatidylcholine, and soybean lecithin are particularly preferable.

In the case of adding such a phospholipid, as for the blending ratio between the PEG-modified phospholipid and the phospholipid in the carrier of the present invention, the phospholipid is appropriately within a range from 0.03 to 100 parts by weight per 1 part by weight of the PEG-modified phospholipid, preferably within a range from 0.05 to 20 parts by weight, more preferably within a range from 0.2 to 1.1 parts by weight.

To the carrier of the present invention, cholesterol can be added other than the PEG-modified phospholipid and Compound A which are the essential components. In the case of adding cholesterol, as for the blending ratio between the PEG-modified phospholipid and cholesterol in the carrier of the present invention, cholesterol is appropriately within a range from 0.01 to 200 parts by weight per 1 part by weight of the PEG-modified phospholipid, preferably within a range from 0.02 to 100 parts by weight.

A dispersion of the carrier of the present invention can be prepared by mixing, for example, a PEG-modified phospholipid and Compound A; a PEG-modified phospholipid, Compound A, and a phospholipid; or a PEG-modified phospholipid, Compound A, and cholesterol, and by dispersing the components in an aqueous solution according to a conventional method. The dispersion procedure can be carried out by an appropriate apparatus such as an ultrasonic dispersion apparatus or an emulsification dispersion apparatus.

III. The composition of the present invention

The particle size of the carrier of the present invention containing a medicine, which carrier is contained in the composition of the present invention, is not particularly limited, and, it is appropriately within a range from, for example, 50 nm to 200 nm, preferably within a range from 60 nm to 150 nm.

Examples of the "medicine" which can be used in the composition of the present invention include water-soluble anionic compounds, antitumor agents, antiviral agents, and antibiotics. Specific examples thereof include nucleic acids such as single-stranded or double-stranded RNAs, single-Stranded or double-stranded DNAs, and oligonucleic acids, acidic sugars such as heparan sulfate and dextran sulfate, cytokines, second messengers such as cyclic AMP, ATP, and IP3, penicillins and cephalosporins, vitamins such as vitamin C and retinols, and other exiting medicines with an acidic group such as interferons (α, β, γ), interleukins (IL-1, IL-2), colony-stimulating factors (CSF), tumor neurosis factors (TNF), levamisol, pestatin, retinoic acid, 5-fluorouracil (5-FU), cytosine arabinoside (Ara-C), adenine arabinoside (Ara-A), cisplatin (CDDP), cyclophosphamide, and azidothymidine (AZT).

Examples of the synthetic double-stranded RNA include those described below.

### 1. Homopolymer-homopolymer complexes

Polyinosinic acid-poiycytidylic acid
Polyinosinic acid-poly(5-bromocytidylic acid)
Polyinosinic acid-poly (2-thiocytidylic acid)
Poly(7-deazainosinic acid)-polycytidylic acid
Poly(7-deazainosinic acid)-poly(5-bromocytidylic acid)
Poly(2'-azidoinosinic acid)-polycytidylic acid
Polyinosinic acid-poly(cytidine-5'-thiophosphoric acid)

### 2. Homopolymer-copolymer complexes

Polyinosinic acid-poly(cytidylic acid, uridylic acid)
Polyinosinic acid-poly(cytidylic acid, 4-thiouridylic acid)

### 3. Complexes of a synthetic nucleic acid and a polycation

Polyinosinic acid-polycytidylic acid-poly-L-lysine

### 4. Others

Polyinosinic acid-poly(1-vinylcytidylic acid)

Examples of the oligonucleic acid include RNA, DNAs and compounds thereof, which have 10 to 3000 nucleobases, preferably 15 to 2000 nucleobases, more preferably 18 to 1000 nucleobases per molecule, for example, siRNAs, miRNAs, shRNAs, non-coding RNAs, antisense DNAs, antisense RNAs, DNA enzymes, ribozymes, and aptamers.

The oligonucleic acid is not limited to naturally types, and at least a part of a sugar, a phosphate backbone or the like constituting a nucleotide thereof may be modified for enhancing the in vino stability such as nuclease resistance. Examples of such a modification include ribose modifications at the 2'-position, ribose modifications at other positions, and phosphate backbone modifications. Examples of the ribose modifications at the 2'-position include modifications by substituting the hydroxyl group at the 2'position of the ribose with H, OR⁵, R⁵, R⁶OR⁵, SH, SR⁵, NH₂, NHR⁵, N (R⁵)₂, N₃, CN, F, Cl, Br, and I. Here, R⁵ represents alkyl or aryl, and R⁶ represents alkylene.

The alkyl of R⁵ is not particularly limited to the form of linear or branched chain, and examples thereof include alkyl having 1 to 6 carbon atoms. Specific examples thereof include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may be substituted with 1 to 3 substituents including, for example, halogen, alkyl, alkoxy, cyano, and nitro. Examples of the halogen include fluorine, chlorine, bromine, and iodine. Examples of the alkyl include the same groups as described in the above alkyl. The alkoxy is not particularly limited to the form of linear or branched chain, and examples thereof include alkoxy having 1 to 6 carbon atoms. Specific examples thereof include methoxy, etuhoxy, n-propoxy, isopropoxy, n-butoxy, isobutoxy, sec-butoxy, tert-butoxy, n-pentyloxy, isopentyloxy, n-hexyloxy, and isohexyloxy. Among these, alkoxy having 1 to 3 carbon atoms is particularly preferable.
Examples of the aryl of R⁵ include aryl having 6 to 10 carbon atoms. Specific examples of the aryl include phenyl, α-naphthyl, and β-naphthyl. Among these, phenyl is particularly preferable.

The alkylene of R⁶ is not particularly limited to the form of linear or branched chain, and examples thereof include alkylene having 1 to 6 carbon atoms. Specific examples thereof include methylene, ethylene, trimethylene, tetramethylene, pentamethylene, hexamethylene, 2-(ethyl)trimethylene, and 1-(methyl)tetramethylene.

Examples of the ribose modifications at other positions include 4'-thio-modifications. Examples of the phosphate backbone modifications include phosphorothioate modifications, phosphorodithioate modifications, alkylphosphonate modifications, and phosphoroamidate modifications.

The weight ratio (the carrier of the present invention/the medicine) of the carrier of the present invention to the medicine to be contained in the composition of the present invention varies depending on the type of the medicine, the blending ratio of the PEG-modified phospholipidor Compound A in the carrier of the present invention, and so on. The weight ratio is appropriately within a range from 0.01 to 1000, preferably within a range from 10 to 300, more preferably within a range from 100 to 200. In the case where the medicine contained therein is an oligonucleic acid, the weight ratio is appropriately within a range from 0.01 to 100, preferably within a range from 1 to 50, more preferably within a range from 5 to 30.

In the composition of the present invention, other than the above-mentioned carrier of the present invention and medicine, a pharmaceutically acceptable addictive can be blended as needed. Examples of the addictive include emulsifying auxiliary agents (such as fatty acids having 6 to 22 carbon atoms and pharmaceutically acceptable salts thereof, albumin, and dextran), stabilizers (such as cholesterol, and phophatidic acid), tonicity agents (such as sodium chloride, glucose, maltose, lactose, sucrose, and trehalose), and pH adjusting agents (such as hydrochloric acid, sulfuric acid, phosphoric acid, acetic acid, sodium hydroxide, potassium hydroxide, and triethanolamine). These can be used singly or in combination of two or more thereof.

The composition of the present invention can be prepared by adding a medicine to a dispersion of the carrier of the present invention and by appropriately stirring the resulting mixture. The composition of the present invention can also be prepared by adding a medicine in the course of producing the carrier of the present invention. The above-mentioned additive can be added at an appropriate time of the process either before or after a dispersion treatment.

The composition of the present invention can be prepared as, for example, a liquid preparation or a lyophilized preparation. In the case of a liquid preparation, the concentration of the carrier of the present invention contained in the composition of the present invention is appropriately within a range from 0.001 w/v% to 50 w/v%, preferably within a range from 0.01 w/v% to 25 w/v%, more preferably within a range from 0.1 w/v% to 10 w/v%.

The lyophilized preparation can be prepared by subjecting the composition of the present invention the form of a liquid preparation to a lyophilization treatment according to a conventional method. For example, the lyophilization treatment can be performed as follows. After the composition of the present invention in the form of a liquid preparation is appropriately sterilized, a given volume thereof is dispensed into a vial, followed by preliminary freezing under conditions of about -40°C to -20°C for about 2 hours. Thereafter, the composition is subjected to primary drying under reduced pressure at about 0°C to 10°C and then to secondary drying under reduced pressure at about 15°C to 25°C. In general, the inside of the vial is replaced with nitrogen gas, and then, the vial is capped, whereby the lyophilized preparation of the composition of the present invention can be prepared.

The lyophilized preparation of the composition of the present invention can generally be used by adding an appropriate solution (solution for re-dissolution) to re-dissolve the preparation. Examples of the solution for re-dissolution include water for injection, physiological saline, and other general infusions. The liquid volume of the solution for re-dissolution varies depending on the use thereof and so on, and is not particularly limited, and the liquid volume of the solution is appropriately 0.5 to 2 times the liquid volume of the composition of the present invention before lyophilization or 500 mL or less.

A disease to which the composition of the present invention can be applied is not particularly limited, and examples thereof include cancer, viral diseases, inflammatory diseases, metabolic diseases, and neurological diseases.

The route of administration of the composition of the present invention is not particularly limited insofar as it is a pharmaceutical acceptable route of administration, and can be selected according to a treatment method. Examples of the route of administration include intravenous administration, intraarterial administration, oral administration, transpulmonary administration, infra-tissue administration, transdermal administration, mucosal administration, intrarectal administration, intrabladder administration, intraperitoneal administration, intraocular administration, intracerebral administration, and intrathoracic administration. Among these, intravenous administration, transdermal administration, and mucosal administration are particularly preferable. The dosage form of the composition of the present invention is not particularly limited, and, examples thereof include various injections, oral agents, infusions, inhalations, eye drops, ointments, lotions and suppositories.

The dose of the composition of the present invention as a medicine is preferably used in consideration of the type and dosage form of the medicine, the patient conditions such as age and body weight, the route of administration, and the nature and severity of the disease. Generally, the dose is within a range from 0.01 mg to 10 g/human/day, preferably within a range from 0.1 mg to 5 g/human/day as the dose of the medicine per adult. In the case where the medicine contained in the composition of the present invention is an oligonucleic acid, generally, the dose of the oligonucleic acid per adult is within a range from 0.1 mg to 10 g/human/day, preferably within a range from 1 mg to 5 g/human/day. The numerical values sometimes vary depending on the type of target disease, the route of administration, and the target molecule. Therefore, in some cases, the dose of the oligonucleic acid may suffice when it is below the range described above. In some cases, a dose above the range described above may be needed. The dose can be administered once daily or in several times a day or can be administered at intervals of one day to several days.

### Examples

Hereinafter, the present invention will be illustrated in more detail with reference to Production Examples, Comparative Examples, and Test Examples. However, the present invention is not limited to the scope described below.

### Production Example_1: Synthesis of oligo_RNA

Using an automatic nucleic acid synthesizer (Expedite 8909, manufactured by Applied BioSystems, Inc.), an oligo RNA having a nucleotide sequence represented by SEQ ID NO: 1, an oligo RNA having a nucleotide sequence represented by SEQ ID NO: 2, an oligo RNA having a nucleotide sequence represented by SEQ ID NO: 3, and an oligo RNA having a nucleotide sequence represented by SEQ ID NO: 4 were synthesized by the amidite method described in the document (Nucleic Acid Research, 1984, Vol. 12, pp. 4539-4557).
The protecting groups of the base moieties were removed by cleavage at CPG using a mixed liquid of concentrated ammonium hydroxide and ethanol (3/1) and further by a reaction in the same solution at 55°C for 18 hours. Subsequently, the silyl group at the 2' -position was deprotected by a reaction at room temperature for 20 hours using a 1 M tetrahydrofuran solution of tetrabutylammonium fluoride. The resulting oligo RNA was purified by reverse-phase chromatography. Further, the dimethoxytrityl group at the 5'-position was deprotected by a reaction at room temperature for 30 minutes using an 80% aqueous solution of acetic acid, and then, the resulting oligo RNA was purified again by ion exchange chromatography. The concentrations of the obtained oligo RNA having a nucleotide sequence represented by SEQ ID NO: 1, oligo RNA having a nucleotide sequence represented by SEQ ID NO: 2, oligo RNA having a nucleotide sequence represented by SEQ ID NO:3, and oligo RNA having a nucleotide sequence represented by SEQ ID NO: 4 were 3.37 mg/mL, 3.45 mg/mL, 10.00 mg/mL, and 10.00 mg/mL, respectively.
Incidentally, it was confirmed by capillary electrophoresis that 90% or more of the obtained oligo RNAs were a full-length RNA.

### Production Example 2: Synthesis of tritium-labeled double-stranded oligo RNA

A tritium-labeled double-stranded oligo RNA comprising an oligo RNA having a nucleotide sequence represented by SEQ ID NO: 1 and an oligo RNA having a nucleotide sequence represented by SEQ ID NO: 2 were synthesized by the incorporation of a tritium-labeled [2,5',8-3H]adenosine 5'-triphosphate ammonium salt (manufactured by Amersham BioSciences, Inc.) using in vitro Transcription T7 Kit (manufactured by Takara-Bio Co., Ltd.).
Subsequently, proteins were removed from the double-stranded oligo RNA using a phenol/chloroform mixed solution, and further, unreacted monomers were removed using a G-25 spin column (manufactured by Pharmacia, Inc.). The concentration of the obtained double-stranded oligo RNA was 4.07 mg/mL. Further, the specific radioactivity thereof was 5.3 x 10⁵ dpm/µg.
Incidentally, it was confirmed by 15 % polyacrylamide electrophoresis that the obtained double-stranded oligo RNA had a chain, length of around 21 base pairs.

### Production Example 3: Synthesis of 1,3-distearoylglycero-2-phosphatidyl-N-(methoxy-polyethylene-glycol-succinyl) eth anolamine

### Step 1: Synthesis of 1,3-distearoylglycerol

Three g of dihydroxyacetone dimer, 22.7 g of stearic acid, 16.8 g of 1-ethyl-3-(3-dimethylaminopropyl) carbodiimide hydrochloride, and 10.8 g of 4-dimethylaminopyridine were stirred in 100 mL of dichloromethane overnight at room temperature. To the reaction solution, 0.5 L of methanol was added, and the resulting powder was recovered by filtration, washed with methanol, and dried. Six g of the obtained powder was suspended in a mixed liquid of 400 mL of tetrahydrofuran and 20 mL of a 10% aqueous solution of acetic acid. To the resulting suspension, 1.1 g of sodium borohydride was added in small portions at 0°C. After the resulting mixture was stirred at room temperature for 6 hours, the reaction solution was poured into a saturated aqueous sodium bicarbonate solution, and an extraction procedure was performed using ethyl acetate. The organic layer was dried and then concentrated under reduced pressure. The resulting residue was purified by silica gel column chromatography, whereby 3.5 g of the intended product was obtained.

### Step 2: Synthesis of diisopropylamine tetrazolide

365 mg of tetrazole was dissolved in 8 mL of acetonitrile, and 1.20 g of diisopropylamine was added dropwise thereto. The resulting mixture was stirred at room temperature for 20 minutes, and the reaction solution was concentrated under reduced pressure, followed by drying, whereby 852 mg of the intended product was obtained.

### Step 3: Synthesis of 1,3-distearoylglycerol 2-O-(2-cyanoethyl N,N-diisopropylphosphoroamidite)

1.39 g of 1,3-distearoylglycerol obtained in the above step 1 was suspended in a mixed liquid of 30 mL of acetonitrile and 10 mL of dichloromethane, and 456 mg of diisopropylamine tetrazolide obtained in the above step 2 and 1 g of bis(N,N-diisopropylamine)cyanoethylphosphite were added thereto, and the resulting mixture was stirred at 40°C for 1.5 hours. The reaction solution was subjected to filtration, and the filtrate was concentrated. The resulting residue was purified by silica gel column chromatography, whereby 800 mg of the intended product was obtained.

³¹P NMR (202 MHz, CDCl₃, δ): 152.283

### Step 4: Synthesis of 1,3-distearoylglycerol2-O-(2-cyanoethyl 2-tert-butoxycarbonylaminoethylphosphate)

700 mg of 1,3-distearoylglycerol 2-O-(2-cyanoethyl N,N-diisopropylphosphoroamidite) obtained in the above step 3, 114 mg of tert-butyl N-(2-hydroxyethyl)carbamate, and 119 mg of tetrazole were dissolved in a mixed liquid of 5 mL of acetonitrile and 5 mL of dichloromethane together with 0.5 g of molecular sieves 4A and the resulting mixture was stirred at room temperature for 30 minutes. To the reaction solution, 20 mL of an iodine solution (0.1 M iodine/tetrahydrofurane: pyridine: water = 7:1:2) was added, and the resulting mixture was further stirred at room temperature for 20 minutes. The reaction solution was subjected to filtration and to the filtrate, a saturated aqueous solution of sodium thiosulfate was added until the color of iodine disappeared. Then, an extraction procedure was performed using ethyl acetate. The organic layer was dried and then concentrated under reduced pressure. The resulting residue was purified by silica gel column chromatography, whereby 700 mg of the intended product was obtained.

³¹P NMR (202 MHz, CDCl₃, δ): 0.12453

MALDI-TOF Mass (m/z) = 923.564 ([M+Na]⁺)

### Step 5: Synthesis of 1,3-distearoylglycerol2-O-(2-aminoethyl phosphate)

15 mL of a mixed liquid of pyridine, triethylamine, and water (3:1:1) was added to 660 mg of 1,3-distearoylgglycerol 2-O-(2-cyanoethyl 2-tert-butoxycarbonylaminoethylphosphate) obtained in the above step 4, and the resulting mixture was stirred at room temperature for 2 hours. After the reaction solution was concentrated under reduced pressure, azeotropic distillation with pyridine was performed three times. Thereafter, azeotropic distillation with dichloromethane was further performed three times. The resulting residue was dissolved in 5 mL of dichloromethane, 5 mL of trifluoroacetic acid was added thereto at 0°C, and the resulting mixture was stirred at room temperature for 30 minutes. After the reaction solution was concentrated under reduced pressure, azeotropic distillation with dichloromethane was performed three times, whereby 410 mg of the intended product was obtained.

³¹P NMR (202 MHz, CDCl₃, δ): -0.0833

### Step 6: Synthesis of 1,3-distearoylglycero-2-phosphatidyl-N-(methoxy-polyethylene glycol succinyl) ethanolamine

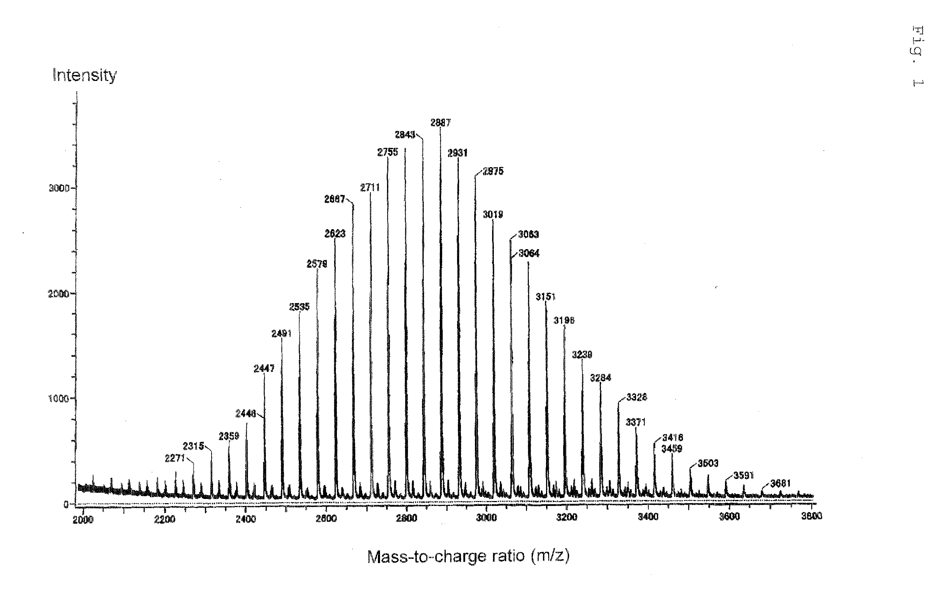

40 mL of dimethoxyethane, 40 mL of dichloromethane, and 10 mL of a saturated aqueous sodium bicarbonate solution were added to 400 mg of 1,3-distearoylglycerol 2-0-(2-aminoethyl phosphate) obtained in the above step 5 and 1.24 g of α-succinimidyloxysuccinyl-ω-methoxy-polyoxyethylene [SUNBRIGHT (registered trademark) ME-020CS, manufactured by NOF Corporation], and the resulting mixture was stirred overnight at room temperature. After water was added to the reaction solution, an extraction procedure was performed 3 times using dichloromethane. The organic layer was dried and then concentrated under reduced pressure. The resulting residue was purified by silica gel column chromatography, whereby 950 mg of the intended product was obtained.
The molecular weight of the obtained product was determined by mass spectrometry using the electrospray ionization method. As a result, as shown in Fig. 1, the obtained product had a molecular weight distribution within a range from 2,000 to 3,800.

### Production Example 4 : Preparation of dispersion of drug carrier

60 mg of Compound A, 8 mg of the PEG-modified phospholipid synthesized in Production Example 3, and 92 mg of 1-palmitoyl -2-oleoyl-sn-glycero-3-phosphocholine (manufactured by NOF Corporation, hereinafter the same is applied) were dissolved in 2 mL of chloroform in a vial, to which nitrogen gas was purged to remove chloroform, thereby forming a thin film on the wall of the vial. After the vial was left to stand overnight under reduced pressure, 1,00 mg of maltose (manufactured by Otsuka Pharmaceutical Co., Ltd.), 4.0 mL of water for injection (manufactured by Otsuka Pharmaceutical Co., Ltd., hereinafter the same is applied), and 81 µL of 1 N hydrochloric acid where added to the vial, and the thin film was dispersed using a vortex mixer. After the dispersion was left to stand at 4°C for 3 hours, sonication was performed for 10 minutes using a microprobe, thereby preparing a dispersion of a drug carrier at 32 mg/mL. Thereafter, the volume of the dispersion was made up to 5.0 mL with water for injection.

### Production Example 5: Preparation of dispersion of drug carrier

A dispersion of a drug carrier was prepared in the same manner as in Production Example 4 using 60 mg of Compound A, 16 mg of the PEG-modified phospholipid synthesized in Production Example 3, and 84 mg of 1-palmitoyl -2-oleoyl-sn-glycero-3-phosphocholine.

### Production Example 6: Preparation of dispersion of drug carrier

A dispersion of a drug carrier was prepared in the same manner as in Production Example 4 using 60 mg of Compound A, 32 mg of the PEG-modified phospholipid synthesized in Production Example 3, and 68 mg of 1-palmitoyl -2-oleoyl-sn-glycero-3-phosphocholine.

### Production Example 7: Preparation of dispersion of drug carrier

A dispersion of a drug carrier was prepared in the same manner as in Production Example 4 using 60 mg of Compound A, 48 mg of the PEG-modified phospholipid synthesized in Production Example 3, and 52 mg of 1-palmitoyl -2-oleoyl-sn-glycero-3-phosphocholine.

### Production Example 8 : Preparation of dispersion of carrier

A dispersion of a drug carrier was prepared in the same manner as in Production Example 4 using 60 mg of Compound A, 64 mg of the PEG-modified phospholipid synthesized in Production Example 3, and 36 mg of 1-palmitoyl -2-oleoyl-sn-glycero-3-phosphocholine.

### Production Example 9: Preparation of dispersion of drug carrier

A dispersion of a drug carrier was prepared in the same manner as in Production Example 4 using 60 mg of Compound A, 80 mg of the PEG-modified phospholipid synthesized in Production Example 3, and 20 mg of 1-palmitoyl -2-oleoyl-sn-glycero-3-phosphocholine.

### Production Example 10: Preparation of dispersion of drug carrier

A dispersion of a drug carrier was prepared in the same manner as in Production Example 4 using 60 mg of Compound A, 88 mg of the PEG-modified phospholipid synthesized in Production Example 3, and 12 mg of 1-palmitoyl -2-oleoyl-sn-glycero-3-phosphocholine.

### Production Example 11: Preparation of dispersion of drug carrier

A dispersion of a drug carrier was prepared in the same manner as in Production Example 4 using 60 mg of Compound A and 100 mg of the PEG-modified phospholipid synthesized in Production Example 3.

### Production Example 12: Preparation of dispersion of drug carrier

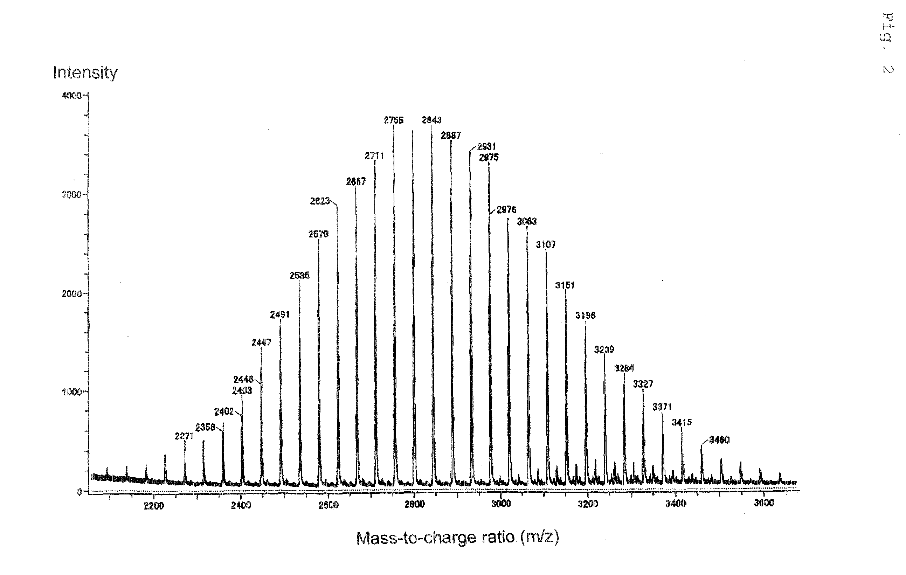

A dispersion of a drug carrier was prepared in the same manner as in Production Example 4 using 60 mg or Compound A, 32 mg of N-(methoxy-polyethylene-glycol-succinyl) distearoylphosphatidylethanolamine [SUNBRIGHT (registered trademark) DSPE-020C, manufactured by NOF Corporation, hereinafter the same is applied] (hereinafter referred to as "Compound B"), and 20 mg of 1-palmitoyl-2-oleoyl-sn-glycero-3-phosphocholine.
Incidentally, the molecular weight of Compound B used was determined by mass spectrometry using the electrospray ionization method. As a result, as shown in Fig. 2, Compound B used had a molecular weight distribution within a range from 2,200 to 3,600.

### Production Example 13: Preparation of dispersion of drug carrier

A dispersion of a drug carrier was prepared in the same manner as in Production Example 4 using 60 mg of Compound A, 48 mg of Compound B, and 52 mg of 1-palmitoyl-2-oleoyl-sn-glycero-3-phosphocholine.

### Production Example 14: Preparation of dispersion of drug carrier

A dispersion of a drug carrier was prepared in the same manner as in Production Example 4 using 60 mg of Compound A, 80 mg of Compound B, and 20 mg of 1-palmitoyl-2-oleoyl-sn-glycero-3-phosphocholine.

### Production Example 15: Preparation of dispersion of drug carrier

A dispersion of a drug carrier was prepared in the same manner as in Production Example 4 using 60 mg of Compound A, 88 mg of Compound B, and 12 mg of 1-palmitoyl-2-oleoyl-sn-glycero-3-phosphochol

### Production Example 16: Preparation of dispersion of drug carrier

A dispersion of a drug carrier was prepared if the same manner as in Production Example 4 using 60 mg of Compound A, 80 mg of the PEG-modified phospholipid synthesized in Production Example 3, and 20 mg of egg yolk lecithin (manufactured by QP Corporation, hereinafter the same is applied).

### Production Example 17: Preparation of pharmaceutical composition

### (1) Preparation of nucleic acid solution

A nucleic acid solution was prepared by mixing 36 µL of the tritium-labeled double-stranded oligo RNA synthesized in Production Example 2, 1.50 mL of the oligo RNA having a nucleotide sequence represented by SEQ ID NO: 1 synthesized in Production Example 1, 1.43 mL of the oligo RNA having a nucleotide sequence represented by SEQ ID NO: 2 synthesized in Production Example 1, and 2.07 mL of water for injection.

### (2) Preparation of pharmaceutical composition

5 mL of the dispersion of a drug carrier prepared in Production Example 4 was added to the total amount of the nucleic acid solution prepared in the above (1), and sonication was performed for 5 minutes. After the resulting solution was centrifuged at 5,000 rpm for 20 minutes and filtered through a 0.22 µm filter, whereby a pharmaceutical composition at 1.0 mg/mL was prepared.

### (3) Measurement of average particle size of drug carrier

The average particle size (volume average) of the drug carrier in the pharmaceutical composition was measured by diluting the composition of the present invention prepared in the above (2) to 0.02 mg/mL with water for injection. Specifically, the average particle size thereof was measured in triplicate using a particle size measurement device [Nicomp C380 (registered trademark) manufactured by Particle Sizing Systems, Inc., hereafter the same is applied] by setting the refractive index to 0.993, the viscosity to 1.333, and the measurement time period to 5 minutes. As a result, the average particle size of the drug carrier in the pharmaceutical composition was 102.7 nm.

### Production Example 18 : Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 5.
Incidentally, the average particle size of the drug carrier in the pharmaceutical composition was measured in the same manner as in Production Example 17 (3) and found to be 104.0 nm.

### Production Example 19: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 6.
Incidentally, the average particle size of the drug carrier in the pharmaceutical composition was measured in the same manner as in Production Example 17 (3) and found to be 103.4 nm.

### Production Example 20: Preparation of pharmaceutical composition

### (1) Preparation of nucleic acid solution

A nucleic acid solution was prepared in the same manner as in Production Example 17(1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 7.
Incidentally, the average particle size of the drug carrier in the pharmaceutical composition was measured in the same manner as in Production Example 17 (3) and found to be 102.5 nm.

### Production Example 21: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 8.
Incidentally, the average particle size of the drug carrier in the pharmaceutical composition was measured in the same manner as in Production Example 17 (3) and found to be 97.9 nm.

### Production Example 22: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 9.
Incidentally, the average particle size of the drug carrier in the pharmaceutical composition was measured in the same manner as in Production; Example 17 (3) and found to be 91.6 nm.

### Production Examples 23: Preparation of Pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 10.
Incidentally, the average particle size of the drug carrier in the pharmaceutical composition was measured in the same manner as in Production Examples 17 (3) and found to be 65.1 nm.

### Production Example 24: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 11.
Incidentally, the average particle size of the drug carrier in the pharmaceutical composition was measured in the same manner as in Production Example 17 (3) and found to be 63.5 nm.

### Production Example 25: Preparation of pharmaceutical composition

### (1) Preparation of nucleic acid solution

A nucleic acid solution was prepared in the same manner as in Production Example 17 (1).

### (2) Preparation of Pharmaceutical composition

A Pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 12.
Incidentally, the average particle size of the drug carrier in the pharmaceutical composition was measured in the same manner as in Production Example 17 (3) and found to be 97.3 nm.

### Production Example 26: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution in the above total amount of the nucleic acid solution prepared in the above (1) and 5 mL of the dispersion of a drug carrier prepared in Production Example 13.
Incidentally, the average particle size of the drug carrier in the pharmaceutical composition was measured in the same manner as in Production Example 17(3) and found to be 97.7 nm.

### Production Example 27: Preparation of Pharmaceutical composition

### (1) Preparation of nucleic acid solution

A nucleic acid solution was prepared in the same manner as in Production Example 17(1).

### (2) Preparation of Pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 14.
Incidentally, the average particle size of the drug carrier in the pharmaceutical composition was measured in the same manner as in Production Example 17 (3) and found to be 98.0 nm.

### Production Example 28: Preparation of pharmaceutical composition

### (1) Preparation of nucleic acid solution

A nucleic acid solution was prepared in the same manner as in Production Example 17 (1) .

### (2) Preparation of pharmaceutical composition

A pharmaceutical composition at 1.0 mg/mL was prepared in the same manner as in Production Example 17(2) using the total amount of the nucleic acid solution prepared in the above (1) and 5 mL of the dispersion of a drug carrier prepared in Production Example 15.
Incidentally, the average particle size of the drug carrier in the Pharmaceutical composition was measured in the same manner as in Production Example 17(3) and found to be 78.2 nm.

### Production Example 29: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 16.
Incidentally, the average particle size of the drug carrier in the pharmaceutical composition was measured in the same manner as in Production Example 17 (3) and found to be 92.7 nm.

### Production Example 30: Preparation of pharmaceutical composition

### (1) Preparation of nucleic acid solution

A nucleic acid solution was prepared in the same manner as in Production Example 17 (1).

### (2) Preparation of pharmaceutical composition

A pharmaceutical composition at 1.0 mg/mL was prepared in the same manner as in Production Example 17 (2) using the total amount of the nucleic acid solution prepared in the above (1) and 5 mL of the dispersion of a drug carrier prepared in Production Example 9.
Incidentally, the average particle size of the drug carrier in the pharmaceutical composition was measured in the same manner as in Production Examples 17(3) and found to be 84.9 nm.

### Production Example 31: Preparation of pharmaceutical composition

### (1) Preparation of nucleic acid solution

A nucleic acid solution was prepared by mixing 0.50 mL of the oligo RNA having a nucleotide sequence represented by SEQ ID NO: 3 synthesized in Production Example 1, 0.50 mL of the oligo RNA having a nucleotide sequence represented by SEQ ID NO: 4 synthesized in Production Example 1, and 4.0 mL of water for injection.

### (2) Preparation of the composition of the present invention

A pharmaceutical composition at 1.0 mg/mL was prepared in the same manner as in Production Example 17 (2) using the total amount of the nucleic acid solution prepared in the above (1) and 5.0 mL of the dispersion of a drug carrier prepared in Production Example 9.
Incidentally, the average particle size of the drug carrier in the pharmaceutical composition was measured in the same manner as in Production Example 17(3) and found to be 95.7 nm.
Further; the double-stranded oligo RNA composed of the oligo RNA having a nucleotide sequence represented by SEQ ID NO: 3 and the oligo RNA having a nucleotide sequence represented by SEQ ID NO: 4 is a double-stranded oligo RNA having an inhibitory activity on the expression of Bcl-2 (see WO 2004/106511).

### Comparative Example 1: Preparation or dispersion of drug carrier as comparative control

A dispersion of a drug carrier as a comparative control was prepared in the same manner as in Production Example 4 using 60 mg of Compound A and 100 mg of egg yolk lecithin.

### Comparative Example 2: Preparation of dispersion of drug carrier as comparative control

A dispersion of a drug carriers as a comparative control was prepared using LIPOFECTIN (registered trademark) (manufactured by Invitrogen, Inc.) by the preparation method instructed by the supply company.

### Comparative Example 3: Preparation of dispersion of drug carrier as comparative control

A dispersion of a drug carrier as a comparative control was prepared using OLIGOFECTAMINE (registered trademark) (manufactured by Invitrogen, Inc.) by the preparation method instructed by the supply company.

### Comparative Example 4: Preparation of Pharmaceutical composition as comparative control

### (1) Preparation of nucleic acid solution

A nucleic acid solution was prepared in the same manner as in Production Example 17 (1).

### (2) Preparation of composition as comparative control

A pharmaceutical composition as a comparative control at 1.0 mg/mL was prepared in the same manner as in Production Example 17 (2) using the total amount of the nucleic acid solution prepared in the above (1) and 5.0 mL of the dispersion of a drug carrier prepared in Comparative Example 1.
Incidentally, the average particle size of the drug carrier in the pharmaceutical composition as a comparative control was measured in the same manner as in Production Example 17 (3) and found to be 148.1 nm.

### Comparative Example 5: Preparation of pharmaceutical composition as comparative control

### (1) Preparation of nucleic acid solution

A nucleic acid solution was prepared in the same manner as in Production Example 17 (1).

### (2) Preparation of pharmaceutical composition as comparative control

A pharmaceutical composition as a comparative control at 1.0 mg/mL was prepared in the same manner as in Production Example 17 (2) using the total amount of the nucleic acid solution prepared in the above (1) and 5.0 mL of the dispersion of a drug carrier prepared in Comparative Example 1.
Incidentally, the average particle size of the drug carrier in the pharmaceutical composition as a comparative control was measured in the same manner as in Production Example 17 (3) and found to be 168.9 nm.

### Comparative Example 6: Preparation of Pharmaceutical composition as Comparative control

### (1) Preparation of nucleic acid solution

A nucleic acid solution was prepared in the same manner as in Production Example 31 (1).

### (2) Preparation of composition as comparative control

A pharmaceutical composition as a comparative control at 1.0 mg/mL was prepared in the same manner as in Production Example 31 (2) using the total amount of the nucleic acid solution prepared the above (1) and 5.0 mL of the dispersion of a drug carrier prepared in Comparative Example 1.
Incidentally, the overage particle size of the drug carrier in the pharmaceutical composition as a comparative control was measured in the same manner as in Production Examples 31 (3) and found to be 138.6 nm.

### Test Example 1: Evaluation of circulating property blood

The circulating property in blood of a drug carrier containing the PEG-modified phospholipid synthesized in Production Example 3 was evaluated using the radioactivity of a nucleic acid contained in the drug carrier as an index.

### (1) Experimental method

Each of the pharmaceutical compositions prepared in Production Examples 17 to 24 was intravenously administered to a male mouse (C576BL/6J, 6 weeks of age, prepared by CLEA Japan, Inc.) through the tail vein at 2.5 mg/kg (nucleic acid content). At 2 hours, 8 hours, and 24 hours after the administration, the whole blood was collected from the abdominal aorta of the mouse under Ethrane anesthesia, and plasma was obtained. Heparin was used as an anticoagulant agent for obtaining plasma. The administration was performed at a dose of 10 mL/kg in all the cases. Four mice were used per group.
10 mL of a scintigraphic agent (Hionic-Fluor, manufactured by PerkinElmer, Inc. hereafter the same is applied) was added to 50 µL of the plasma and mixed with each other, and then, the radioactivity of each sample was measured using a liquid scintillation counter. From the results, the distribution ratio (% of dose) of the drug carrier was calculated.

### (2) Experimental results

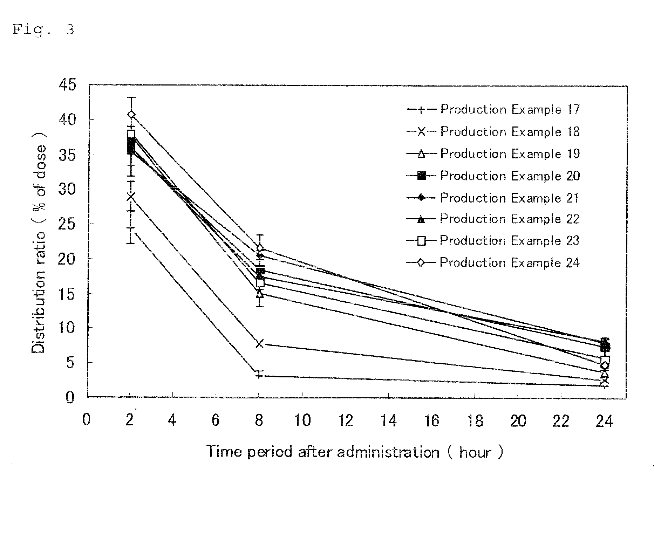

As shown in Figs. 3 and 4, the drug carrier had the longest circulation time when the content of the PEG-modified phospholipid of Production Example 3 was within a range from 30 wt% to 50 wt%.

### Test Example 2: Evaluation of circulating property in blood

The circulating property in blood of a drug carrier containing Compound B was evaluated using the radioactivity of a nucleic acid contained in the drug carrier as an index.

### (1) Experimental method

Each of the pharmaceutical compositions prepared in Production Examples 25 to 28 was intravenously administered to a male mouse (C57BL/6J, 6 weeks of age, prepared by CLEA Japan, Inc.) through the tail vein at 2.5 mg/kg (nucleic acid content). At 24 hours after the administration, the whole blood was collected from the abdominal aorta of the mouse under Ethrane anesthesia, and plasma was obtained. Heparin was used as an anticoagulant agent for obtaining plasma. The administration performed at a dose of 10 mL/kg in all the cases. Four mice were used per group.
10 mL of a scintigraphic agent was added to 50 µL of the plasma and mixed with each other, and then, the radioactivity of each sample was measured using a liquid scintillation counter. From the results, the distribution ratio (% of dose) of the drug carrier was calculated.

### (2) Experimental results

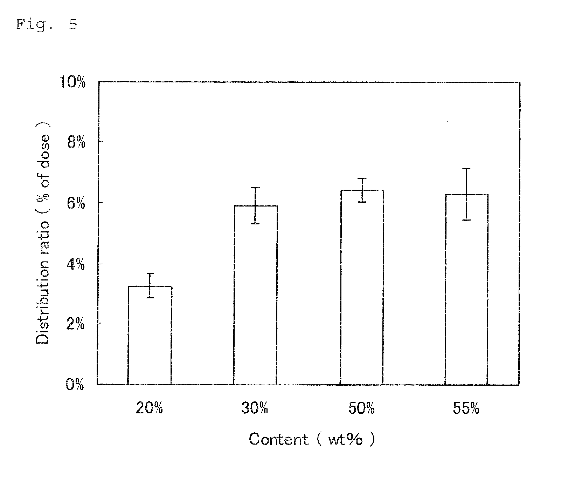

As shown in Fig 5, the drug carrier had a long circulation time when the content of Compound B was 30 wt% or more.

### Test Example 3: Evaluation of biodistribution of the carrier of the present invention

The biodistribution of the carrier of the present invention was evaluated using the radioactivity of a nucleic acid contained in the drug carrier as an index.

### (1) Experimental method

The pharmaceutical composition prepared in Production Example 29 or Comparative Example 4 was intravenously administered to a male mouse (C57BL/6J, 6 weeks of age, prepared by CLEA Japan, Inc.) through the tail vein at 2.5 mg/kg (nucleic acid content). At 5 minutes, 15 minutes, 30 minutes, 1 hour, 2 hours, 4 hours, 8 hours, 24 hours, 48 hours, and 72 hours after the administration, the whole blood was collected from the abdominal aorta of the mouse under Ethrane anesthesia, and plasma was obtained. Heparin was used as an anticoagulant agent for obtaining plasma. The administration was performed at a dose of 10 mL/kg in all the cases. Three or four mice were used per group.
10 mL of a scintigraphic agent was added to 50 µL of the plasma and mixed with each other, and then, the radioactivity of each sample was measured using a liquid scintillation counter. From the results, the distribution ratio (% of dose), distribution, volume (L/kg), elimination half-life (hours), area under the plasma concentration (µg·hours/mL), and clearance (L/hours·kg) of the drug carrier in the plasma were calculated.

### (2) Experimental results

### (i) Plasma concentration

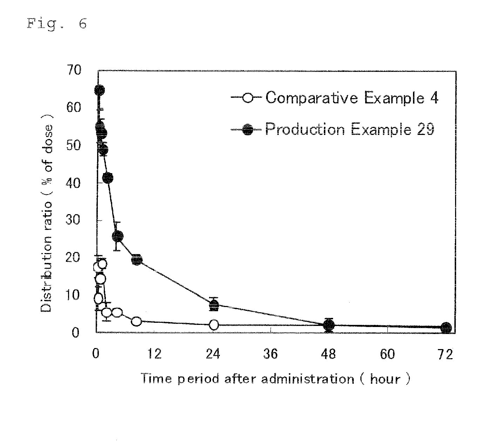

As shown in Fig 6, the carrier of the present invention in the composition of the present invention of Production Example 29 had a longer circulation time in plasma than the drug carrier as a comparative control in the pharmaceutical composition as a comparative control of Comparative Example 4.

### (ii) Pharmacokinetic parameter

As shown in Table 1, the area under the plasma concentration (AUC₀₋₈) of the carrier of the present invention in the composition of the present invention of Production Example 29 was about 5 times higher than that of the drug carrier as a comparative control in the pharmaceutical composition as a comparative control of Comparative Example 4. Further, the distribution volume of the carrier of the present invention was about one-eighth of that of the drug carrier as a comparative control.

**[Table 1]**

| | | Production Examples 29 | Comparative Example 4 |
|---|---|---|---|
| Distribution volume | Vd | 0.17 | 1.40 |
| Elimination half-life | T_{½, α} | 4.69 | 3.43 |
| | T_{½, β} | 21.3 | 81.2 |
| Area under the plasma concentration | AUC_{0-∞} | 345.4 | 170.0 |
| | AUC₀₋₈ | 132.1 | 28.3 |
| Total clearance | CLₜₒₜ | 0.0072 | 0.0147 |

### Test Example 4: Evaluation of biodistribution of the carrier of the present invention

The biodistribution of the carrier of the present invention was evaluated using the radioactivity of a nucleic acid contained the drug carrier as an index.

### (1) Experimental method

The pharmaceutical composition prepared in Production Example 29 or Comparative Example 4 was intravenously administered to a male mouse (C57BL/6J, 6 weeks of age, prepared by CLEA Japan, Inc.) through the tail vein at 2.5 mg/ (nucleic acid content). At 30 minutes, 2 hours, 8 hours, and 24 hours after the administration, the whole blood was collected from the abdominal aorta of the mouse under Ethrane anesthesia, and plasma was obtained. Heparin was used as an anticoagulant agent for obtaining plasma. Further, concurrently with the blood collection, the liver, lung, spleen, and kidney were resected and the wet weights thereof were weighed, respectively. The administration was performed at a dose of 10 mL/kg in all the cases. Three or four mice were used per group.
Each of the organs was dissolved in a vial by adding 1 mL of a tissue solubilizer (SOLVABLE, manufactured by PerkinElmer, Inc.) and shaking the resulting mixture at 40°C for two nights.
10 mL of a scintigraphic agent was added to a sample in which 50 to 200 mg of each organ was dissolved and mixed with each other, and then, the radioactivity of each sample was measured using a liquid scintillation counter. From the results, the distribution ratio (% of dose) of the drug carrier in each organ was calculated. Incidentally, the distribution ratio (% of dose) of the drug carrier in plasma was calculated in the same manner as in Test Example 1.

### (2) Experimental results

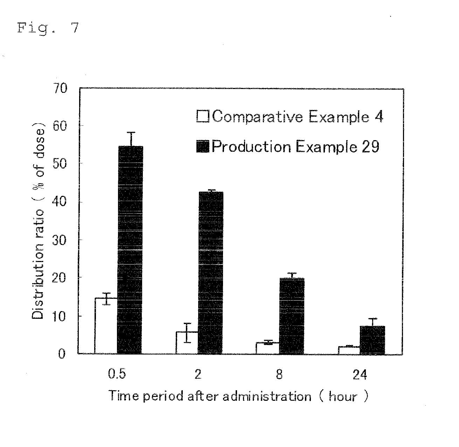

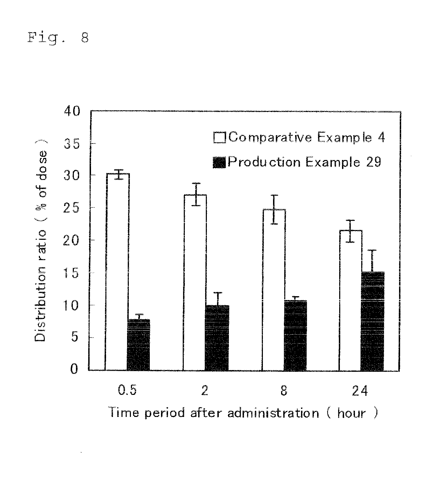

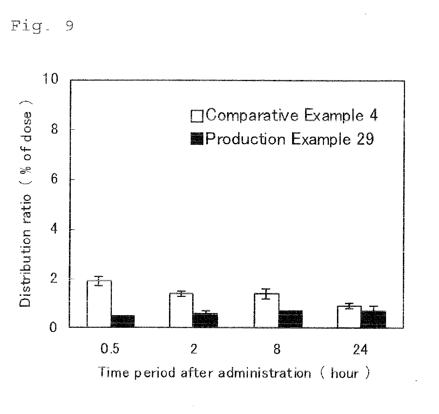

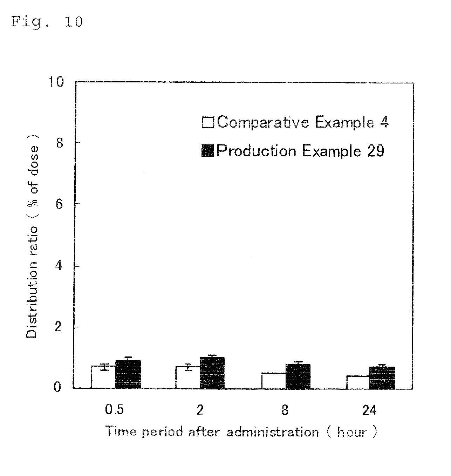

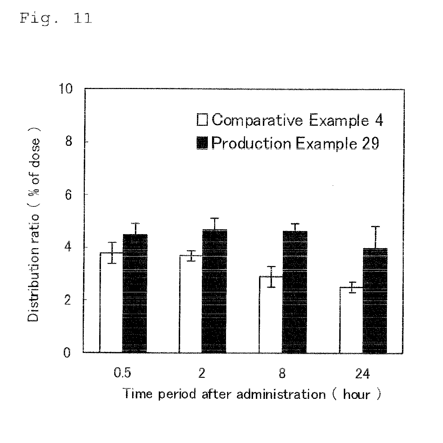

As shown in Fig 7, the plasma distribution ratio of the carrier of the present invention in the composition of the present invention of Production Example 29 was higher than that of the drug carrier as a comparative control in the pharmaceutical composition as a comparative control of Comparative Example 4. As shown in Figs. 8 and 9, the liver and spleen distribution ratio of the carrier of the present invention in the composition of the present invention of Production Example 29 was lower than that of the drug carrier as a comparative control in the pharmaceutical composition as a comparative control of Comparative Example 4. As shown in Figs. 10 and 11, there was no difference in the distribution ratio in the lung and kidney between the drug carriers in the pharmaceutical compositions of Production Example 29 and Comparative Example 4.

### Test Examples Evaluation of biodistribution of the carrier of the present invention in mouse implanted with cancer cells

The biodistribution of the carriers of the present invention in a mouse implanted with cancer cells was evaluated using the radioactivity of a nucleic acid contained in the drug carrier as an index.

### (1) Implantation mouse model of cancer cells

A mouse implanted with cancer cells was prepared by subcutaneously implanting 1 x 10⁶ A431 cells (human squamous epithelial cells) in a male nude mouse (BALB/cA Jcl-nu, 9 weeks of age, prepared by CLEA Japan, Inc.) and rearing the mouse for 9 days after the implantation.

### (2) Experimental method

The pharmaceutical composition prepared in Production Example 30 or Comparative Example 5 was intravenously administered to the mouse prepared in the above (1) through the tail vein at 10 mg/kg (nucleic acid content). At 8 hours, 24 hours, and 72 hours after the administration, the whole blood was collected from the abdominal aorta of the mouse under Ethrane anesthesia, and plasma was obtained. Heparin was used as an anticoagulant agent for obtaining plasma. Further, concurrently with the blood collection, the peripheral tissues of cancer and cancerous nodes where resented and the wet weight thereof were weighed, respectively. The administration was performed at a dose of 10 mL/kg in all the cases. Three mice were used per group.
The amounts (µg/g or µg/mL) of the delivered drug carrier, in the peripheral tissues of cancer, cancerous nodes, and plasma were measured in the same manner as in Test Example 1 or 4.

### (3) Experimental results

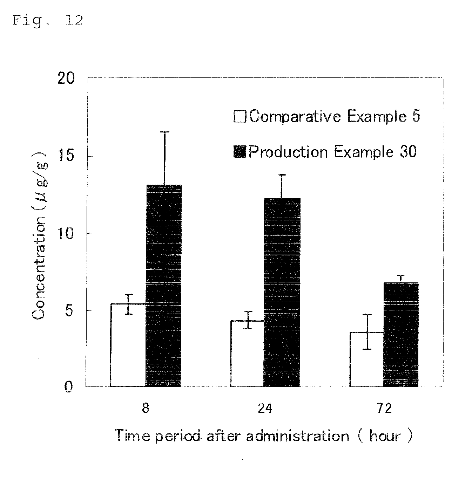

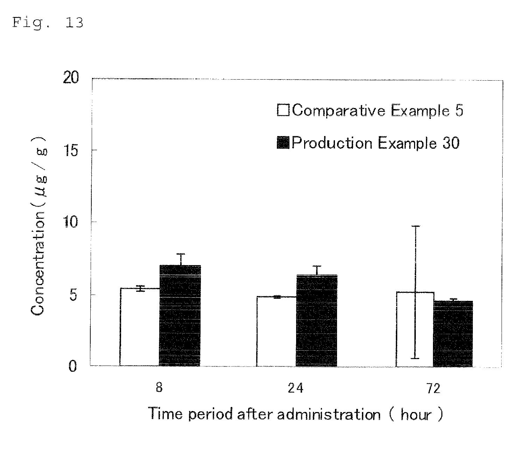

As shown in Fig 12, the amount of the delivered carrier of the present invention in the composition of the present invention of Production Example 30 in the peripheral tissues of cancer was higher than that of the drug carrier as a comparative control in the pharmaceutical composition as a comparative control of Comparative Example 5. On the other hand, as shown in Fig. 13, there was no difference in the distribution ratio the cancerous nodes between the drug carriers in the pharmaceutical compositions of Production Example 30 and Comparative Example 5. Incidentally, as shown in Fig. 14, the distribution ratio of the carrier of the present invention in the composition of the present invention of Production Example 30 in the plasma was higher than that of the drug carrier as a comparative control in the pharmaceutical composition as a comparative control of Comparative Example

### Test Example 6: Evaluation of hemolytic property of the carrier of the present invention

### (1) Experimental method

The blood collected from a male rat (Slc:SD, 7 weeks of age, prepared by Japan SLC, Inc.) was centrifuged at 3,000 rpm for 10 minutes, and then the upper layer was removed, whereby a erythrocyte suspension was obtained. To the obtained erythrocyte suspension, physiological saline for injection (manufactured by Otsuka Pharmaceutical Factory, Inc., hereafter the same is applied) was added in an amount twice that of the erythrocyte suspension and mixed with each other. Then, the resulting mixture was centrifuged at 3,000 rpm for 5 minutes. This procedure was repeated two more times. The obtained erythrocyte suspension, was diluted to 1 x 10⁹ cells/mL with physiological saline for injection.
Each of the dispersion of a drug carrier prepared in Production Example 16 and Comparative Examples 1 to 3 was diluted with 10 % maltose to a desired concentration within a range from 0.3 µg/µL to 30 mg/µL. After 285 µL of the diluted dispersion of a drug carrier was preincubated at 37°C for 10 minutes, 15 µL of the erythrocyte suspension was added thereto and mixed with each other, and the resulting mixture was incubated at 37°C for 30 minutes. The reaction solution was centrifuged at 3,000 rpm for 3 minutes and the supernatant was recovered. The absorbance of the supernatant was measured at 405 nm.
The degree of hemolysis was calculated by taking the absorbance obtained in the case where the drug carrier was not added as 0% hemolysis and the absorbance obtained in the case where 0.02% Triton X100 was added as 100% hemolysis. Further, from the calculated degree of hemolysis, the concentration causing 50% hemolysis was calculated.

### (2) Experimental results

As shown in Table 2, the concentration of the carrier of the present invention of Production Example 16 causing 50% hemolysis was higher than those of the drug carriers as comparative controls of Comparative Examples 1 to 3.

**[Table 2]**

| | Concentration causing 50% hemolysis (µg/mL) |
|---|---|
| Production Example 16 | 11900 |
| Comparative Example 1 | 50.8 |
| Comparative Example 2 | 1.0 |
| Comparative Example 3 | 1.6 |

### Test Example 7: Evaluation of cytotoxicity of the carrier of the present invention

### (1) Experimental method

Human umbilical vein endothelial cells (manufactured by Sanko Junyaku Co., Ltd.) were seeded in a 96-well plate at 3,000 cells/well and were cultured overnight. Each of the dispersions of a drug carrier prepared in Production Example 16 and Comparative Examples 1 to 3 was diluted with 10% maltose to a desired concentration within a range from 3 µg/µL to 10 mg/µL. The diluted dispersion of a drug carrier-was added to each well in an amount one-tenth of the volume of the culture medium therein. After culturing for 72 hours, viable cells were counted using Cell Counting Kit-8 (WST-8, manufactured by Dojin Chemical Co., Ltd.), and from the obtained value, a 50% cell growth inhibitory concentration was calculated. Incidentally, in the culture of human umbilical vein endothelial cells, an M199 culture medium (manufactured by Nissui Pharmaceutical Co., Ltd.) was used.

### (2) Experimental results

As shown in Table 3, the 50% cell growth inhibitory concentration of the carrier of the present invention of Production Example 16 was higher than those of the drug carriers as comparative controls of Comparative Examples 1 to 3.

**[Table3]**

| | 50% cell growth inhibitory concentration (µg/mL) |
|---|---|
| Production Example 16 | 738.1 |
| Comparative Example 1 | 151.1 |
| Comparative Example 2 | 6.7 |
| Comparative Example 3 | 15.2 |

### Test Example 8: Evaluation of cytokine inducibility of the composition of the present invention

### (1) Experimental method

Human fresh blood was collected, and in order to prevent coagulation, HEPARIN SODIUM INJECTION (manufactured by Ajinomoto Co., Ltd.) was mixel therein in an amount of 1 mL per 10 mL of the blood. An equal volume of phosphate buffered saline (hereinafter referred to as "PBS") was added thereto, and the blood in an amount of 10 mL per 3 mL of Ficoll-Paque PLUS (manufactured by GE Healthcare BioSciences, Inc.) was overlaid carefully thereon so as not to disturb the interface. Then, centrifugation was performed at 400 x for 30 minutes at room temperature, whereby peripheral blood mononuclear cells were obtained. The obtained peripheral blood mononuclear cells were washed twice with PBS and then suspended in an RPMI 1640 culture medium (manufactured by Nissui Pharmaceutical Co., Ltd.) containing 10 % bovine fetal serum (manufactured by JRH BioSciences, Inc.), 100 U/mL pencillin (manufactured by Nacalai Tesque, Inc.), and 100 µg/mL streptomycin (manufactured by Nacalai Tesque, Inc.). Then, the cells therein were counted and a cell suspension at 2 x 10⁶ cells was prepared.
The cell suspension prepared was seeded in a 48-welt plate at 300 µL (6x 10⁵ cells) /well, and the cells were cultured under conditions of 37°C and 5% CO₂ for 3 hours. Then, each of the pharmaceutical compositions prepared in Production Examples 17 to 24 and 31 and Comparative Example 6 was added to the culture medium to a desired concentration (Production Examples 17 to 24: 100 nM, Production Example 31 and Comparative Example 6: 30, 100, and 300 nM). The cells were cultured for 24 hours after the addition of the pharmaceutical composition, and then, the culture supernatant was recovered and an ELISA was performed. IFN-α was measured, using human interferon-α ELISA Kit (manufactured by Biosource, Inc.) according to the protocol attached thereto.

### (2) Experimental results,

As shown in Table 4, the IFN-α inducibility of the pharmaceutical compositions of Production Examples 17 to 19 was higher than that of the pharmaceutical compositions of Production Examples 20 to 24.
As shown in Table 5, the level of IFN-α induced by the treatment with the composition of the present invention of Production. Example 31 was lower than that induced by the treatment with the pharmaceutical composition as a comparative control of Comparative Example 6.
Incidentally, n.d. in Tables 4 and 5 indicates a level below the detection limit.

**[Table 4]**

| | Concentration of IFN-α(pg/mL) |
|---|---|
| Production Example 17 | 310 |
| Production Example 18 | 494 |
| Production Example 19 | 431 |
| Production Example 20 | 25 |
| Production Example 21 | n.d. |
| Production Example 22 | n.d. |
| Production Example 23 | n.d. |
| Production Example 24 | n.d. |

**[Table 5]**

| | Treatment concentration | Concentration of IFN-α (pg/mL) |
|---|---|---|
| Production Example 31 | 30 nM | n.d. |
| | 100 nM | n.d. |
| | 300 nM | n.d. |
| Comparative Example 6 | 30 nM | 58 |
| | 100 nm | 230 |
| | 300 nM | 1000 |

### Test Example 9: Evaluation of drug efficacy of the composition of the present invention

### (1) Implantation mouse model of cancer cells

A mouse lasted with cancer cells was prepared by subcutaneously implanting 3 x 10⁵ PC-3 cells (human prostate cancer cells) in a male nude mouse (BALB/cA Jcl-nu, 6 weeks of age, prepared by CLEA Japan, Inc.).

### (2) Experimental method

The composition of the present invention prepared in Production Example 31 was intravenously administered to the mouse prepared in the above (1) through the tail vein at 10 mg/kg (nucleic acid content) once daily for 5 consecutive days starting from 10 days after the implantation. Further, the composition of the present invention was administered in the same manner for 5 consecutive days starting from 17 days after the implantation. The administration was performed at a dose of 10 mL/kg in all the cases. Six mice were used per group. Incidentally, a mouse with the administration of 10% maltose was used as a negative control.
The tumor volume was determined by measuring the major and minor axes of a tumor after 10, 13, 17, 20, and 24 days from the implantation and making a calculation according to the formula: [(minor axis)² (major axes) / 2].

### (3) Experimental results

As shown in Fig. 15, by administering the composition of the present invention prepared in Production Example 31, an increase in the tumor volume was suppressed.

### Test Example 10: Evaluation of drug efficacy of the composition of the present invention

### (1) Implantation mouse model of cancer cells

A mouse implanted with cancer cells was prepared by intraperitoneally implanting 7 x 10⁵ MCAS cells (human ovarian cancer cells) in a female nude mouse (BALB/cA Jcl-nu, 6 weeks of age, prepared by CLEA Japan, Inc.).

### (2) Experimental method

Evaluation was performed for the case where the composition of the present invention was continuously administered and the case where the composition of the present invention was intermittently administered. In the continuous administration schedule, the composition of the present invention prepared in Production Example 31 was administered once daily on day 3 to day 7 and day 10 to day 14 after the implantation. In the intermittent administration schedule, the composition of the present invention prepared in Production Example 31 was administered once daily on day 4, 7, 11, 14, 18, 21, 25, 28, 32, and 35 after the implantation. The composition of the present invention was intravenously administered through the tail vein at 10 mg/kg (nucleic acid content). The administration was performed at a dose of 10 mL/kg in all the cases. Six mice were used per group. Incidentally, a mouse with the administration of 10% maltose was used as a negative control.

### (3) Experimental results

As shown in Figs. 16 and 17, by administering the composition of the present invention prepared in Production Example 31, the survival rate was prolonged.

### Test Example 11: Evaluation of drug efficacy of the composition of the present invention

### (1) Preparation of mouse bearing liver metastases of cancer cells

A mouse bearing liver metastases of cancer cells was prepared by implanting 1 x 10⁶ cells of A549 cell (human non-small-cell lung cancer) in the spleen of a male nude mouse (BALB,/cA Jcl-nu, 6 weeks of age, prepared by CLEA Japan, Inc.) and extirpating the spleen at 10 minutes after the implantation.

### (2) Experimental method

The composition of the present invention prepared in Production Example 31 was intravenously administered to the mouse prepared in the above (1) through the tail vein at 10 mg/kg (nucleic acid concentration) once daily for 5 consecutive days starting from 7 days after the implantation. Further, the composition of the present invention was administered in the same manner for 5 consecutive days starting from 14 days after the implantation. The administration was performed at a dose of 10 mL/kg in all the cases. Six mice were used per group. Incidentally, a mouse with the administration of 10% maltose was used as a negative control.

### (3) Experimental results

As shown in Fig. 18, by administering the composition of the present invention prepared it Production Example 31, the survival rate was prolonged.

### Test 12; Evacuation of drug of the composition of the present invention

### (1) Implantation mouse model of cancer cells

A mouse implanted with cancer cells was prepared by implanting 1 x 10⁶ cells of HPAC cell (human pancreatic cancer cells) the pancreas of a male nude mouse (BALB/cA Jcl-nu, 7 weeks of age, prepared by CLEA Japan, Inc.). A mouse treated in the same manner using a culture medium in place of the HPAC cells was used as a sham-operated group.

### (2) Experimental method

The composition of the present invention prepared in Production Example 31 was intravenously administered to the mouse prepared in the above (1) through the tail vein at 10 mg/kg (nucleic acid content) once daily on day 6 to day 10 and day 13 to day 17 after the implantation. After 29 days from the implantation, the mouse was dissected, the weight of the pancreas was measured, and the antitumor effect was evaluated. Incidentally, 8 mice were used per group. Further, a mouse with the administration. of 10% maltose was used as a negative control.

### (3) Experimental results

As shown in Fig. 19, by administering the composition of the present invention prepared in Production Example 31, an increase in the weight of the pancreas was suppressed.

## Claims

1. A long-circulating drug carrier, comprising a polyethylene glycol-modified phospholipid represented by the following general formula (I) or a pharmaceutically acceptable salt thereof: 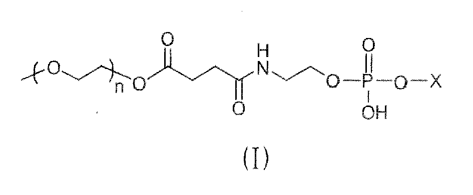 [wherein X represents the following (II) or (III); and n represents an integer of 30 to 150], 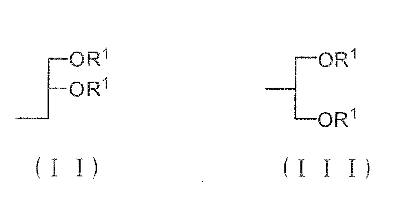 [wherein R¹ represents a saturated linear fatty acid residue having 17 to 22 carbon atoms], and 2-0- (2-diethylaminoethyl) carbamoyl-1,3-O-dioleoylglylcerol, wherein the polyethylene glycol-modified phospholipid represented by the general formula (I) is contained in an amount within a range from 30 wt% to 50 wt% in the total weight of the lipids in the drug carrier.

2. The long-circulating drug carrier according to claim 1, wherein the polyethylene glycol-modified phospholipid is 1,3-distearoyldlycero-2-phosphatidyl-N-(methoxy-polyethylene-g lycol-succinyl)ethanolamine or N-(methoxy-polyethylene-glycol-succinyl)distearoylphosphatidylethanolamine.

3. The long-circulating drug carrier according to any one of claims 1 and 2, further comprising a phospholipid.

4. A pharmaceutical composition, comprising the long-circulating drug carrier according to any one of claims 1 to 3, which incorporates a medicine.

5. The pharmaceutical composition according to claim 4, wherein the medicine is a single-stranded or double-stranded RNA, a single-stranded or double-stranded RNA, an oligonucleic acid, or a water-soluble anionic compound.

6. The pharmaceutical composition according to claim 5, wherein the oligonucleic acid is a short interfering RNA, a microRNA, a short hairpin RNA, an antisense DNA, an antisense RNA, a DNA enzyme, a ribozyme, an aptamer or non-coding RNA.


SEQUENCE LISTING

<110> Nippon Shinyaku Co., Ltd.

<120> Drug carrier

<130> S3363 EP S3

<140> EP 08 79 1872.8
<141> 2008-07-30

<150> JP 2008-160231
<151> 2008-06-19

<160> 4

<210> 1
<211> 21
<212> DNA
<213> Artificial sequence

<220>
<221> source
<223> /note="Description of artificial sequence: synthesized DNA/RNA;
a strand concerning Preparation Examples 1 and 2, wherein 2 nucleotides
of 3'-terminus are constructed with DNA, and the rest are RNA."

<400> 1
gcuaugaaac gauaugggct t                                                21

<210> 2
<211> 21
<212> DNA
<213> Artificial sequence

<220>
<221> source
<223> /note="Description of artificial sequence: synthesized DNA/RNA;
a strand concerning Preparation Examples 1 and 2, wherein 2 nucleotides
of 3'-terminus are constructed with DNA, and the rest are RNA."

<400> 2
gcccauaucg uuucauagct t                                                21

<210> 3
<211> 21
<212> DNA
<213> Artificial sequence

<220>
<221> source
<223> /note="Description of artificial sequence: synthesized DNA/RNA;
a strand concerning Preparation Example 1, wherein 2 nucleotides
of 3'-terminus are constructed with DNA, and the rest are RNA."

<400> 3
gugaugaagu acauccauut t                                                21

<210> 4

<211> 21
<212> DNA
<213> Artificial sequence

<220>
<221> source
<223> /note="Description of artifical sequence: synthesized DNA/RNA;
      a strand concerning Preparation Example 1, wherein 2 nucleotides
      of 3'-terminus are constructed with DNA, and the rest are RNA."

<400> 4
aauggaugua cuucaucact t                                              21